# EUROPÄISCHE PATENTSCHRIFT

(11) **EP 0 651 635 B1**
(45) Veröffentlichungstag und Bekanntmachung des Hinweises auf die Patenterteilung: **23.04.1997**
(21) Anmeldenummer: 93915645.1
(22) Anmeldetag: 17.07.1993
(51) Int. Cl.: A61K 9/70

(54) **WIRKSTOFFPFLASTER FÜR NIEDRIGSCHMELZENDE UND/ODER FLÜCHTIGE WIRKSTOFFE**
PATCH FOR LOW-MELTING AND/OR VOLATILE ACTIVE SUBSTANCES
EMPLATRE MEDICAMENTEUX POUR PRINCIPES ACTIFS A BAS POINT DE FUSION ET/OU VOLATILS

(30) Priorität: 23.07.1992 DE 4224325
(43) Veröffentlichungstag der Anmeldung: 10.05.1995
(73) Patentinhaber: SCHWARZ PHARMA AG, D-40789 Monheim/Rhld. (DE)
(72) Erfinder: MERKLE, Hans Peter, CH-8049 Zürich (CH); NAGELS, Klaus, D-53115 Bonn (DE); SCHACHT, Dietrich, D-50935 Köln (DE); WOLFF, Hans-Michael, D-40789 Monheim (DE)
(86) Internationale Anmeldenummer: DE9300638
(87) Internationale Veröffentlichungsnummer: WO9402123

(56) Entgegenhaltungen:
- EP-A- 0 186 019
- EP-A- 0 305 757
- EP-A- 0 439 180

## Beschreibung

Die vorliegende Erfindung betrifft ein Wirkstoffpflaster zur kontrollierten topischen oder transdermalen Abgabe von flüchtigen Wirkstoffen, bestehend aus einer Rückschicht bzw. Trägerschicht und einer damit verbundenen Reservoirschicht aus einem SEBS-Dreiblockcopolymeren, die zugleich als Haft- und Steuerschicht für die Wirkstoffabgabe dient, und Verfahren zur Herstellung dieses Wirkstoffpflasters aus der Schmelze. Das Hautpflaster ist mit einer Schutzfolie abgedeckt, die vor Gebrauch des Pflasters, d.h. vor Anbringen desselben auf die Haut, durch Abziehen von der Reservoirschicht entfernt wird.

Wirkstoffpflaster, die eine kontrollierte Abgabe des oder der wirksamen Bestandteile an die Haut ermöglichen, sind aus der Literatur bereits bekannt. Ausführungsformen solcher Pflaster, bei denen der Wirkstoff in einer dünnen Haftkleberschicht gelöst oder homogen dispergiert ist und diffusionskontrolliert freigesetzt wird, stellen konzeptionell einfache, für die serienmäßige Produktion prinzipiell geeignete transdermale oder topische Systeme dar.

In der Praxis ist die Entwicklung und/oder Herstellung solcher Wirkstoffpflaster jedoch häufig mit den im Folgenden auswahlsweise genannten Nachteilen verbunden und gestaltet sich dadurch entsprechend aufwendig:
- Die Klebeeigenschaften der Reservoirschicht können bei hoher Wirkstoffbeladung nicht optimal eingestellt werden, so daß das Pflaster mit einer zusätzlichen Haftschicht ausgestattet werden muß, um einerseits während der Anwendung eine gute Adhäsion auf der Hautoberfläche zu erzielen und andererseits ein vollständiges, schmerzfreies Abziehen des Pflasters von der Haut nach Gebrauch zu ermöglichen.
- Das Reservoir muß mehrschichtig aufgebaut werden, um ausreichende Mengen an Wirkstoff in das Pflaster einarbeiten zu können und/oder sind zusätzliche, räumlich und funktionell von der Haftschicht getrennte Depots einzurichten.
- Um eine kontrollierte, kontinuierliche Wirkstofffreisetzung über längere Applikationszeiträume aufrechtzuerhalten und/oder bei der Wirkstoffabgaberate pro Zeiteinheit Hautirritationen und/oder systemische Nebenwirkungen zumindest zu begrenzen, ist eine zusätzliche Steuerschicht erforderlich.
- Die haftklebende Reservoirschicht wird aus der Lösung hergestellt, so daß das Problem des Absaugens von Lösemittelresten und ggf. auch der damit erfolgenden Abdampfung von flüchtigen Wirkstoffen auftritt. Die Verwendung von Lösemitteln bei der Herstellung von wirkstoffbeladenen Haftkleberschichten ist aus mehreren Gründen nachteilig. Die Herstellung der Lösungen erfordert mindestens einen technisch aufwendigen Verfahrensschritt. Für medizinische Zwecke müssen hochreine und damit teure Lösemittel für die Auflösung der Kleber bzw. deren Ausgangsmaterialien eingesetzt werden, um eine entsprechende Rückstandsfreiheit im haftklebenden Reservoir sicherzustellen. Ein weiteres Problem besteht darin, Lösemittelfreiheit im Pflaster zu erreichen. Hierfür sind technisch aufwendige Trocknungsstrecken und Absauganlagen erforderlich. Zusätzlich müssen Wiedergewinnung und Abscheidung der Lösemittel technisch gewährleistet sein, um Umweltbelastung zu vermeiden; daneben stellt die Brennbarkeit der Lösemittel ein zusätzliches Risiko dar. Die meisten organischen Lösemittel sind ferner schädlich für den menschlichen Organismus, so daß aufwendige Schutzmaßnahmen für die im Betrieb tätigen Personen getroffen werden müssen.

Aus EP 0144486 sind Hautpflaster,u.a. auch für die transdermale Applikation von Bupranolol, bekannt, die den Wirkstoff in einem mehrschichtig aufgebauten Reservoir enthalten, wobei als Steuerungselement für die Wirkstoffabgabe zusätzlich ein mehrstufiges Wirkstoffkonzentrationsgefälle von der äußeren, d.h. von der der Trägerfolie zugekehrten Reservoirschicht zur Haut hin vorgesehen ist.

US 4,668,232 beschreibt ebenfalls u.a. Wirkstoffpflaster mit β-Blockern, bei denen ein haftklebendes, bupranolol- oder propranololhaltiges Reservoir in zwei Teilschritten aufgebaut wird, wobei in diesem Fall dem Reservoir zur Verbesserung resp. Steuerung seiner Wirkstofffreisetzungseigenschaften in Wasser quellfähige Polymere zugesetzt werden.

Aus EP 0186071 sind transdermale Abgabesysteme mit dem β-Blocker Timolol bekannt, die aus Gründen der lokalen Verträglichkeit die Wirkstofffreisetzung aus dem Reservoir auf max. 20 µg/cm²/h mittels diskreter Steuerschichten begrenzen.

Die mit dem Einsatz von Lösemitteln in der Wirkstoffpflasterentwicklung und -herstellung verbundenen Nachteile sind durch Fertigung selbstklebender Wirkstoffreservoire aus der Schmelze zu umgehen. So sind z.B. aus der US 4,485,077 mit Indomethacin beladene Haftschmelzkleber bekannt und in der JP 63203616 sind Haftschmelzkleber für Pflaster und dergleichen, insbesondere für Etofenamat, beschrieben. Gemäß DE-P 37 43 947 eignen sich die beiden vorgeschlagenen Anwendungsformen für Haftschmelzkleber mit hohen Verarbeitungstemperaturen, jedoch nicht für niedrigschmelzende und/oder flüchtige Wirkstoffe, wie beispielsweise das empfindliche Nikotin mit sehr niedrigem Siedepunkt und hohen Verdampfungsraten. Die DE-P 37 43 947 beschreibt entsprechend ein Verfahren, bei dem das Nikotin-Reservoir unter Verwendung eines Haftschmelzklebers mit einer Verarbeitungstemperatur von 40 - 80°C hergestellt wird. Es werden verschiedene Nikotin-Pflaster mit und ohne ein räumlich und funktionell von der Haftschicht getrenntes Nikotin-Depot beschrieben. Beispiele, aus denen die Wirkstoffbeladung des Haftschmelzklebers bei einschichtigen Systemen entnommen werden kann, oder Angaben zur Beladungskapazität solcher Kleberformulierungen, sind in der genannten Anmeldung nicht enthalten. Vielmehr ist beschrieben, daß die dort genannten Vorrichtungen auch ein oder mehrere Nikotin-Depots, in dem/denen Nikotin mit einer gegenüber der nikotinaufweisenden Haftschmelzklebstoffschicht erhöhten Konzentration(en) vorliegt, besitzen, wodurch höhere Dosen des Nikotins verarbeitet werden können und damit die Vorrichtung länger im Einsatz bleiben kann, bevor sie ausgewechselt werden muß. Der Einbau von zusätzlichen Depots in ein Pflaster bedingt einen zusätzlichen technischen Aufwand und infolgedessen verteuern sich somit Entwicklung und Herstellung.

EP 0 521 761 offenbart einen speziell die Wundheilung fördernden Verband, der aus einer Kunststoffmatrix besteht, die von einer Mischung aus Blockcopolymeren des Typs S-EB-S mit Weichmachern gebildet wird.

Der erfinderische Gedanke ist, einen derartigen Matrixwundverband zur Verfügung zu stellen, der die Wunde gegenüber der äußeren Umgebung schützt und die Wundausschwitzungen zurückhält, dabei jedoch in der Lage ist, ein feuchtes Milieu zu gewährleisten. Dieses soll derart sein, daß es vorteilhaft für das Wachstum und die zelluläre Vervielfältigung ist und eben nicht an der Wunde anhaftet mit der Folge, daß eine Verletzung des Hauttraumas beim Entfernen des Verbandes vermieden wird und gleichwohl unter guten Bedingungen die Deckgewebebildung begünstigt wird.

Wenngleich hier Blockcopolymere des Typs S-EB-S erwähnt werden, werden sie immer in Mischung mit Weichmachern genannt und diese ausschließlich als Adhäsivmaterial verwendet.

Schließlich wird andeutungsweise erwähnt, daß die beanspruchte Masse auch in therapeutisch wirksamer Menge pharmazeutisch wirksame Stoffe enthalten könnte. Die Wirkstoffbeladung des Klebers, Angaben, welche Wirkstoffe oder Wirkstoffgruppen verwendbar sind oder Daten zur Beladungskapazität derartiger Kleberformulierungen fehlen jedoch.

EP 0 356 382 beschreibt ein mehrschichtiges Pflaster, dessen Wirkstoff-abgabefähige Reservoirschicht aus einer Mischung von Styrol-Blockmischpolymerisaten mit Alkan- oder Alkadienhomopolymeren besteht. Zusätzlich muß diese Reservoirschicht jedoch mindestens ein die Hautpermeabilität von Wirkstoffen förderndes Mittel enthalten. Gegebenenfalls müssen weitere Steuerungsmittel, wie eine Membran vorhanden sein.

Aus den vorgenannten Gründen ist ein solcher Pflasteraufbau nicht nur technisch schwierig umzusetzen, sondern wegen den notwendigerweise enthaltenden Hautpenetrationsförderern unerwünscht.

Die europäische Offenlegungsschrift EP 0 249 979 offenbart schließlich einen Hot-melt-Kleber der Typen A-B-A (Dreiblockcopolymer) oder A-B-A-B-A-B (Multiblockcopolymere), die zur Verwendung von Absorptionsvorrichtungen geeignet sind, die an Geweben befestigt werden sollen. Beispielhaft werden als derartige Vorrichtungen Sanitätsbinden oder Windeln genannt. Für diesen Verwendungszweck sind die oben erwähnten Coblockpolymere weiters notwendigerweise mit einer Vielzahl von Zusatzstoffen versetzt.

Ein Hinweis, daß diese Klebertypen pharmazeutische Wirkstoffe enthalten, ist diesem Dokument nicht zu entnehmen. Eine Erwähnung, daß die Klebertypen für pharmazeutische Wirkstoffe als Haft- und Steuerschicht dienen könnten, fehlt.

EP 0 186 019 beschreibt ein wirkstoffhaltiges Pflastersystem, das in Wasser quellbare, im Klebefilm nicht lösliche Polymere enthält. Der Zusatz dieser speziellen quellbaren Polymere gewährleistet eine reproduzierbare, über den gesamten Applikationszeitraum kontrollierte Wirkstoffabgabe in hoher, therapeutisch sinnvoller Wirkstoffmenge.

Teilweise wird auch die Verwendung eines Dreiblock-Polystyrol-Poly(ethylen-butylen)-Polystyrol-Copolymers vom SEBS-Typ beschrieben. Jedoch wird dieses Polymer ausschließlich in organischen Lösemitteln in notwendiger Zumischung der in Wasser quellbaren Polymere verwendet. Nach der Lehre dieses Dokuments ist nur die Kombination, bestehend aus dieser Klebermasse, den quellbaren Polymeren und organischen Lösungsmitteln in der Lage, ein Wirkstoffpflaster bereitzustellen, das eine reproduzierbare, über den gesamten Applikationszeitraum möglichst kontrollierte Wirkstoffabgabe in insgesamt hoher Wirkstoffmenge gewährleistet.

Ein Hinweis, daß der Kleber vom SEBS-Typ zur Herstellung von therapeutisch verwendbaren Wirkstoffpflastern allein einsetzbar ist, ohne jeglichen Zusatz weiterer, die Beladung mit Wirkstoff und die aus dem Pflaster abgebende Wirkstoffmenge regelnde Stoffe, läßt sich diesem Dokument nicht entnehmen.

Schließlich beschreibt die EP 0 439 180 ein transdermales therapeutisches System mit dem Wirkstoff Tulobuterol. In diesem Dokument werden Styrol-1,3-Dien-Styrol-Blockcopolymere als Polymercomponente eingesetzt. Dem Dokument ist zu entnehmen, daß sich dieses Polymer lediglich für die galenische Zubereitungsform eines Pflasters mit dem Wirkstoff Tulobuterol, jedoch schon nicht mehr für den der gleichen Wirkstoffklasse, den β-Sympathomimetika, angehörenden Wirkstoff Salbutamol geeignet ist.

Weiterhin ist das vorgenannte Blockcopolymer ein Elastomer, das chemisch ungesättigte Gruppen als Baubestandteile enthält und somit gegen Oxidation und Abbau durch Scherbeanspruchung bereits während der Verarbeitung zu schützen ist. Hinzu kommen Schutzmaßnahmen im fertigen Pflaster als Arzneimittel während der Lagerung und Einschränkungen in der Verwendung von Schutzfolien. Der Patient verlangt transparente Folien, die während der Applikationsdauer dem Licht bzw. mit Sterilisationsmitteln versehenem Badewasser, z.B. Chlor oder Ozon ausgesetzt werden können.

Das Dokument beschreibt jedoch nicht, daß Copolymere vom SEBS-Typ, die im Mittelblock chemisch gesättigte Gruppen als Baubestandteile enthalten, allgemein in Wirkstoffpflastern mit der Funktion der gleichzeitigen Haft- und Steuerschicht für die Wirkstoffabgabe unter Vermeidung der vorgenannten Nachteile verwendet werden können, wenn sie, wie nachfolgend beschrieben, erfindungsgemäß verwendet werden.
Aufgabe der vorliegenden Erfindung ist es daher, die vorgenannten Nachteile der gattungsgemäßen Hautpflaster zur topischen und/oder transdermalen Applikation von niedrigschmelzenden und/oder flüchtigen Wirkstoffen, insbesondere von Nikotin und von β-Rezeptorenblockern wie z.B. Bupranolol, zu vermeiden. Überraschenderweise wurde gefunden, daß ein Wirkstoffpflaster zur kontrollierten Abgabe von Wirkstoffen an die Haut, bestehend aus einer Rückschicht, einem damit verbundenen, wasserunlöslichen Klebefilm aus einem wirkstoffhaltigen Haftschmelzkleber, als Matrixschicht ausgestaltet, und einer den Klebefilm abdekkenden, wieder ablösbaren Schicht, dadurch gekennzeichnet, daß die Matrixschicht einen Haftschmelzkleber ohne Zusatz von in Wasser quellbaren Polymeren, der ein chemisch gesättigtes Dreiblockcopolymer aus Polystyrolblock-copoly(ethylen-butylen)-block-poly-styrol (SEBS) ist, in einer Konzentration von 10 bis 80 Gew.-% und einen bei der Verarbeitungstemperatur des Haftschmelzklebers flüssigen, niedrigschmelzenden und/oder flüchtigen Wirkstoff in einer Konzentration von 2,5 bis 25 Gew.-% enthält sowie ggf. Klebrigmacher, die Beladungskapazität der Reservoirschicht erhöht, ohne zusätzliche Depots und Steuerelemente und/oder Steuerschichten sowie kein Lösemittel beinhaltet. Bevorzugt beträgt der Styrolgehalt des SEBS-Dreiblockcopolymeren 10 bis 50 Gew.-% und besonders bevorzugt 10 bis 30 Gew.-%.

In dem Klebefilm der erfindungsgemäßen Wirkstoffpflaster sind ferner bevorzugt zwischen 20 und 90 Gew.-%, ganz besonders bevorzugt 40 bis 70 Gew.-%, Klebrigmacher ( Tackifier ) sowie gegebenenfalls 0,1 bis zu 1 % Alterungsschutzmittel enthalten. Bevorzugte Tackifier sind aliphatische und/oder aromatische Kohlenwasserstoffharze, die mit den Endblöcken und/oder Mittelblock des SEBS-Polymeren kompatibel sind. Ferner wird bzw. werden bevorzugt Hydroabiethylalkohol und/oder dessen Derivate als Tackifier eingesetzt.

Als Alterungsschutzmittel können Antioxidantien wie Tocopherol, substituierte Phenole, Hydrochinone, Brenzkatechine und aromatische Amine verwendet werden.

Das erfindungsgemäße Wirkstoffpflaster kann hergestellt werden, indem die Bestandteile des Haftschmelzklebers vor Zugabe des Wirkstoffes unter Erwärmung auf 100 bis 200°C, bevorzugt 110 bis 170°C, in einer Inertgasatmosphäre bis zum Erhalt einer homogenen Schmelze gemischt werden und der Wirkstoff anschließend unter Inertgas in der Schmelze des Haftklebers bei einer Verarbeitungstemperatur von 100 bis 200°C, bevorzugt 110 bis 130°C, aufgelöst wird. Bevorzugt wird die homogene, wirkstoffhaltige Haftschmelzklebermasse auf die wieder ablösbare Schutzschicht oder eine antiadhäsive Grundlage durch Extrusion, Gießen, Walzenauftrag, Rakelauftrag, Aufsprühen oder ein Druckverfahren aufgebracht und mit der Rückschicht abgedeckt. Eine andere Vorgehensweise besteht darin, die homogene, wirkstoffhaltige Haftschmelzklebermasse auf die Rückschicht durch Extrusion, Gießen, Walzenauftrag, Rakelauftrag, Aufsprühen oder ein Druckverfahren aufzubringen und mit der wieder ablösbaren Schutzschicht abzudecken. Bevorzugt erfolgt die Vereinzelung der Wirkstoffpflaster durch Schneiden und/oder Formatstanzen.

Weiterhin hat sich überraschend gezeigt, daß SEBS-Dreiblockcopolymere mit niedrigschmelzenden und/oder flüchtigen Wirkstoffen, wie z.B. Nikotin und Bupranolol, Reservoirschichten bilden, die
1. aus der Schmelze bei Verarbeitungstemperaturen oberhalb 100°C ohne Zersetzung des Wirkstoffes und/oder des Polymeren hergestellt werden können,
2. ohne Verlust ihrer Kohäsivität und Klebkraft große Menge an Wirkstoff aufnehmen können, so daß sich der Einbau zusätzlicher Depots und/oder wirkstoffbindender, in der Haftklebermasse unlöslicher Substanzen erübrigt,
   und
3. bei denen die Wirkstofffreisetung ohne zusätzliche Steuerschichten durch den Styrolgehalt des SEBS-Dreiblockcopolymeren und/oder durch Einsatz von Tackifiern, die mit den Endblöcken und/oder dem Mittelblock des SEBS-Blockcopolymeren kompatibel sind, auf die erforderliche Rate eingestellt werden kann.

Überraschenderweise werden ferner bei Gewinnung des erfindungsgemäßen, SEBS-basierten Wirkstoffreservoirs aus der Schmelze höhere Freisetzungsraten des Pflasters erzielt als bei Herstellung aus der Lösung, wodurch der Wirkstoffanteil im Reservoir gesenkt werden kann, ohne daß die Freigabekapazität des Pflasters gegenüber entsprechend aufgebauten und zusammengesetzten lösungsmittelbasierten Systemen erniedrigt wird. Technischer Aufwand und infolge davon können die Kosten des Pflasters so durch die Einsparung von Lösemitteln, zusätzlicher Reservoir- und Steuerschichten sowie Wirkstoff niedrig gehalten werden.Die Erfindung wird anhand folgender Beispiele erläutert:

### Beipiele 1a bis 1f

### Herstellung nach dem Hot melt-Verfahren

Kraton G 1657 (SEBS-Dreiblockcopolymer), Regalrez 1094 (aliphatisches Kohlenwasserstoffharz), Abitol (Hydroabietylalkohol) und Irganox 1010 (Antioxidans) werden in einem Laborkneter bei 110 -150°C in der angegebenen Menge (s. Tab.1) unter Argon aufgeschmolzen und bis zur Homogenität gemischt (Dauer ca 60 Minuten). In der klaren Schmelze werden 23,9 g Bupranolol unter Argon bei 140°C gelöst (Dauer ca 20 Minuten). Die so erhaltene bupranololhaltige Haftschmelzklebmasse wird in einer kühlbaren, antiadhäsiv beschichteten Form zu einem ca 250 µm dicken Film ausgegossen, innerhalb von 5 Minuten auf 12 - 14°C abgekühlt und mit einer 70 µm dicken Polyesterfolie (Rückschicht) abgedeckt. Die offene Klebefläche des so erhaltenen, aus Klebefilm und Rückschicht bestehenden Laminats wird auf eine beidseitig mit Silikon beschichtete 100 µm dicke Polyesterfolie (= wieder ablösbare Schutzschicht) kaschiert.

Danach werden Einzelpflaster mit einer Fläche von 8 cm² ausgestanzt.

### Vergleichsbeispiele 1a' bis 1f'

### Herstellung nach dem Lösungsmittelverfahren

Die in Tabelle 1 aufgeführten Komponenten inkl. Bupranolol werden in einen Jodzahlkolben eingewogen und unter Schütteln in einer Mischung aus 50 ml Petroleumbenzin und 15 ml Toluol gelöst. Die lösungsmittelhaltige Masse wird mit einem Streichrakel auf eine 100 µm dicke Polyesterfolie ausgestrichen und 3 Tage bei 25°C im Umlufttockenschrank getrocknet, so daß ein Klebefilm von ca 174 g/m² resultiert. Die offene Klebefläche des so erhaltenen aus Klebefilm und Rückschicht bestehenden Laminats wird auf eine beidseitig mit Silikon beschichtete 100 µm dicke Polyesterfolie (= wieder ablösbare Schutzschicht) kaschiert.

Danach werden Einzelpflaster mit einer Fläche von 8 cm² ausgestanzt.

**Tabelle 1**

| **Zusammensetzung der erfindungsgemäßen Hot melt - Pflaster und Vergleichsbeispiele** | | | | | | |
|---|---|---|---|---|---|---|
| Beispiel (Vergleichsbeispiel) | Mengenangaben in g * 10⁻¹ bzw. in g für Vergleichsbeispiele | | | | | |
| | Kraton GX # 1657 | Regalrez # 1094 | Abitol | Irganox # 1010 | Bupranolol | Gesamt |
| 1a (1a') | 8,57 | 7,50 | 5,36 | 0,10 | 2,39 | 23,93 |
| 1b (1b') | 6,97 | 9,11 | 5,36 | 0,10 | 2,39 | 23,93 |
| 1c (1c') | 5,36 | 10,72 | 5,36 | 0,10 | 2,39 | 23,93 |
| 1d (1d') | 5,36 | 9,11 | 6,97 | 0,10 | 2,39 | 23,93 |
| 1e (1e') | 5,36 | 7,50 | 8,57 | 0,10 | 2,39 | 23,93 |
| 1f (1f') | 6,97 | 7,50 | 6,97 | 0,10 | 2,39 | 23,93 |

### Wirkstofffreisetzung

Zur Messung der Wirkstofffreisetzung werden 8 cm² große Pflasterabschnitte verwendet.

Die Prüfung wird nach der Paddle-Over-Disk-Methode gemäß USP XXII in 600 ml Phosphatpuffer pH 5.5 als Freisetzungsmedium durchgeführt, Probenentnahmen erfolgen alle 15 Minuten. Der Bupranololgehalt in den Probelösungen wird flüssigketschromatographisch bestimmt.

Die Ergebnisse der Wirkstofffreisetzung nach 2, 4, 6, 8, 12 und 24 Std. sind für die Beispiele 1a bis 1f in Tabelle 2a sowie für die entsprechenden Vergleichsbeispiele in Tabelle 2b zusammengefaßt.

**Tabelle 2a**

| **Wirkstofffreisetzung (Hot melt - Pflaster)** | | | | | | |
|---|---|---|---|---|---|---|
| Beispiel | Mittlere Freisetzung in mg/8 cm² nach | | | | | |
| | 2 h | 4 h | 6 h | 8 h | 12 h | 24 h |
| # 1a | 1.62 | 2,28 | 2,79 | 3,22 | 3,92 | 5,53 |
| # 1b | 1,20 | 1,67 | 2,02 | 2,31 | 2,80 | 3,91 |
| # 1c | 0,67 | 0,92 | 1,08 | 1,22 | 1,47 | 1,99 |
| # 1d | 0,87 | 1,18 | 1,41 | 1,60 | 1,92 | 2,65 |
| # 1e | 1,07 | 1,52 | 1,84 | 2,10 | 2,54 | 3,49 |
| # 1f | 1,29 | 1,79 | 2,18 | 2,50 | 3,02 | 4,17 |

**Tabelle 2b**

| **Wirkstofffreisetzung (Vergleichsbeispiele)** | | | | | | |
|---|---|---|---|---|---|---|
| Vergleichsbeispiel | Mittlere Freisetzung in mg/8 cm² nach | | | | | |
| | 2 h | 4 h | 6 h | 8 h | 12 h | 24 h |
| # 1a' | 1,43 | 1,99 | 2,44 | 2,81 | 3,41 | 4,78 |
| # 1b' | 0,82 | 1,23 | 1,56 | 1,84 | 2,31 | 3,35 |
| # 1c' | 0,35 | 0,44 | 0,51 | 0,56 | 0,67 | 1,01 |
| # 1d' | 0,76 | 1,04 | 1,24 | 1,41 | 1,69 | 2,26 |
| # 1e' | 0,72 | 1,03 | 1,28 | 1,50 | 1,88 | 2,80 |
| # 1f' | 1,10 | 1,58 | 1,93 | 2,22 | 2,72 | 3,95 |

Wie der Vergleich der Meßreihen in Tabelle 2a und 2b zeigt, liegen die Freisetzungsraten der Hot melt - Pflaster bei gleicher Zusammensetzung und Wirkstoffkonzentration der Haftklebemasse trotz völliger Lösemittelfreiheit überraschenderweise zu allen Meßzeitpunkten, z.T. deutlich, über denjenigen der lösungsmitttelbasierten Systeme.

### Beispiele 2a bis 2f

### Herstellung nach dem Hot melt - Verfahren

Kraton G 1657 (SEBS-Dreiblockcopolymer ), Regalrez 1094 (aliphatisches Kohlenwasserstoffharz), Kristalex F 85 (aromatisches Kohlenwasserstoffharz), Abitol (Tackifier) und Irganox 1010 (Antioxidans) werden in einem Laborkneter bei 110 - 150°C in der angegebenen Menge (s. Tab.3) unter Argon aufgeschmolzen und bis zur Homogenität gemischt (Dauer ca 60 Minuten). In der klaren Schmelze wird das Bupranolol in der angegebenen Menge unter Argon bei 140°C gelöst (Dauer ca 20 Minuten). Die so erhaltene bupranololhaltige Haftschmelzklebmasse wird in einer beheizten, wasserkühlbaren, antiadhäsiv beschichteten Form zu einem ca 250 µm dicken Film ausgegossen, innerhalb von 5 Minuten auf 12 - 14°C abgekühlt und mit einer 70 µm dicken Polyesterfolie (Rückschicht) abgedeckt. Die offene Klebefläche des so erhaltenen aus Klebefilm und Rückschicht bestehenden Laminats wird auf eine beidseitig mit Silikon beschichtete 100 µm dicke Polyesterfolie (= wieder ablösbare Schutzschicht) kaschiert.

Danach werden Einzelpflaster mit einer Fläche von 8 cm² ausgestanzt.

**Tabelle 3**

| **Zusammensetzung Beispiel 2, Endblockharzmodifizierte Rezepturen (Hot melt - Pflaster)** Mengenangaben in g | | | | | | | |
|---|---|---|---|---|---|---|---|
| Beispiel | Kraton Regalrez Kristalex Abitol Irganox Bupranolol Gesamt GX | | | | | | |
| | #1657 | #1094 | #F85 | | #1010 | | |
| 2a | 60,00 | 75,00 | 15,00 | 41,00 | 0,88 | 21,32 | 213,20 |
| 2b | 48,75 | 86,25 | 15,00 | 41,00 | 0,88 | 21,32 | 213,20 |
| 2c | 37,50 | 97,50 | 15,00 | 41,00 | 0,88 | 21,32 | 213,20 |
| 2d | 37,50 | 86,25 | 26,25 | 41,00 | 0,88 | 21,32 | 213,20 |
| 2e | 37,50 | 75,00 | 37,50 | 41,00 | 0,88 | 21,32 | 213,20 |
| 2f | 48,75 | 75,00 | 26,25 | 41,00 | 0,88 | 21,32 | 213,20 |

### Wirkstofffreisetzung

Zur Messung der Wirkstofffreisetzung werden 8 cm² große Pflasterabschnitte verwendet. Die Prüfung wird nach der Paddle-Over-Disk-Methode wie für Beispiel 1 beschrieben durchgeführt. Die Ergebnisse der Wirkstofffreisetzung nach 2, 4, 6, 8, 12 und 24 Std. sind für die Beispiele 2a bis 2f in Tabelle 4 zusammengefaßt.

**Tabelle 4**

| **Wirkstofffreisetzung Beispiel 2, Endblockharzmodifizierte Rezepturen (Hot melt - Pflaster).** | | | | | | |
|---|---|---|---|---|---|---|
| Beispiel | mg/8 cm² | | | | | |
| | 2h | 4h | 6h | 8h | 12h | 24h |
| #2a | 1,06 | 1,47 | 1,79 | 2,06 | 2,51 | 3,55 |
| #2b | 0,74 | 1,04 | 1,26 | 1,45 | 1,77 | 2,46 |
| #2c | 0,44 | 0,61 | 0,74 | 0,84 | 1,01 | 1,37 |
| #2d | 0,50 | 0,70 | 0,85 | 0,98 | 1,19 | 1,67 |
| #2e | 0,60 | 0,84 | 1,02 | 1,18 | 1,43 | 2,01 |
| #2f | 0,80 | 1,14 | 1,39 | 1,60 | 1,96 | 2,77 |

Wie die in Tabelle 4 dargestellten Ergebnisse zeigen, läßt sich die freigegebene Wirkstoffmenge mit Hilfe von aromatischen Kohlenwasserstoffharzen retardieren. Die Veränderung der Anteile von SEBS Polymer, aliphatischen und aromatischen Kohlenwasserstoffharzen ist in der Art möglich, dass ein erforderliches Freigabemuster eingehalten werden kann, ohne zusätzliche Steuermembranen einbauen zu müssen.

Gleichzeitig lassen sich so erforderliche Rezeptureinstellungen mit Blick auf Klebeleistung, Wasserdampfdurchlässigkeit und hautverträglichem Freigabeverhaltens erreichen, ohne die Wirkstoffbeladung verändern zu müssen.

### Beispiele 3a bis 3e und 4a bis 4e

### Herstellung nach dem Hotmeltverfahren

Kraton G 1657 (SEBS-Dreiblockcopolymer ) oder Cariflex TR 1107 (SIS-Dreiblockcopolymer), Regalrez 1094 (aliphatisches Kohlenwasserstoffharz), Abitol (Tackifier)und Irganox 1010 (Antioxidans) werden in einem Laborkneter bei 110-150°C in der angegebenen Menge (s. Tab. 5) unter Argon aufgeschmolzen und bis zur Homogenität gemischt (Dauer ca 60 Minuten). Die Klebemasse wird ausgegossen und auf 4°C abgekühlt.

Ein Teil der hergestellten Klebemasse wird im Laborkneter bei 110-150°C aufgeschmolzen (Dauer ca 10 min). Die Klebemasse wird mit Abitol verdünnt, so dass die in Tabelle 6 angegebene Zusammensetzung quantitativ erreicht wird. Der klaren Schmelze werden die in Tabelle 6 und 7 angegebenen Bupranolol-Mengen zugesetzt und unter Argon bei 140 °C gelöst. (Dauer ca 20 Min). Die so erhaltene bupranololfreie oder bupranololhaltige Haftschmelzklebmasse wird in einer beheizten, wasserkühlbaren, antiadhäsiv beschichteten Form zu einem ca 250 µm dicken Film ausgegossen, innerhalb von 5 Min. auf 12-14°C abgekühlt und mit einer 70 µm dicken Polyesterfolie (Rückschicht) abgedeckt. Die offene Klebefläche des so erhaltenen aus Klebefilm und Rückschicht bestehenden Laminats wird auf eine beidseitig mit Silikon beschichteten100 µm dicke Polyesterfolie (= wiederablösbare Schutzschicht) kaschiert.

Danach werden Einzelpflaster mit einer Fläche von 8 cm² ausgestanzt.

**Tabelle 5**

| **Zusammensetzung Beispiel 3, gesättigter Mittelblock (Hotmeltpflaster).** Mengenangaben in g | | | | | | |
|---|---|---|---|---|---|---|
| Beispiel | Kraton GX Cariflex TR Regalrez Abitol Irganox Gesamt | | | | | |
| | #1657 | #1107 | #1094 | | #1010 | |
| 3, a-e | 58,30 | | 79,58 | 61,20 | 0,92 | 200 |
| 4, a-e | | 58,30 | 79,58 | 61,20 | 0,92 | 200 |

**Tabelle 6**

| **Zusammensetsung Beispiel 3, gesättigter Mittelblock (Hotmeltpflaster).** Mengenangaben in g | | | | | | |
|---|---|---|---|---|---|---|
| Beispiel | Kraton Regalrez Abitol Irganox Bupranolol Gesamt GX | | | | | |
| | #1657 | #1094 | | #1010 | | |
| 3a | 29,17 | 39,83 | 30,54 | 0,46 | 0 | 100 |
| 3b | 28,42 | 38,80 | 29,83 | 0,45 | 2,50 | 100 |
| 3c | 27,69 | 37,80 | 29,07 | 0,44 | 5,00 | 100 |
| 3d | 26,23 | 35,81 | 27,54 | 0,41 | 10,00 | 100 |
| 3e | 23,32 | 31,81 | 24,48 | 0,36 | 20,00 | 100 |

**Tabelle 7**

| **Zusammensetzung Beispiel 4, ungesättigter Mittelblock (Hotmeltpflaster).** Mengenangaben in g | | | | | | |
|---|---|---|---|---|---|---|
| Beispiel | Cariflex Regalrez Abitol Irganox Bupranolol Gesamt TR | | | | | |
| | #1107 | #1094 | | #1010 | | |
| 4a | 29,17 | 39,83 | 30,54 | 0,46 | 0 | 100 |
| 4b | 28,42 | 38,80 | 29,83 | 0,45 | 2,50 | 100 |
| 4c | 27,69 | 37,80 | 29,07 | 0,44 | 5,00 | 100 |
| 4d | 26,23 | 35,81 | 27,54 | 0,41 | 10,00 | 100 |
| 4e | 23,32 | 31,81 | 24,48 | 0,36 | 20,00 | 100 |

### Dynamisch-mechanische Analyse

### Charakterisierung des Mittelblock-Temperaturbereiches

Die wirkstofffreien bzw. wirkstoffbeladenen Klebemassen wurden mit Hilfe der dynamisch-mechanischen Analyse charakterisiert. Die Wirkstoffbeladung betrug 2,5, 5, 10 und 20% Bupranolol.

Die Bestimmung des dynamisch-mechanischen Verhaltens im Temperaturbereich des Mittelblock-Glasüberganges erfolgte mit einem Rheometrics RDS 7700. Als Steuergerät wurde ein PC verwendet, der mit der Software RHIOS 3.01 betrieben wurde. Es wurde im Parallel-Platten-Modus gearbeitet. Der Plattendurchmesser betrug 8 mm. Die Frequenz der sinusförmigen Anregung betrug 1 Hz bzw. 6.28 rad/s. Der erfasste Temperaturbereich lag zwischen - 10 und 35°C. Die Temperatur wurde in Schritten von 4° C erniedrigt. Ausgangstemperatur war 35°C. Die Temperaturausgleichszeit der Probe betrug 120 s. Der Tangens delta (Dämpfung), das Maximum des Tangens delta und die Temperatur des Maximums, der Verlust- und Schubmodul wurden bestimmt.

**Tabelle 8**

| **Temperatur beim Maximum des Tangens delta bei wirkstofffreien und wirkstoffbeladenen Klebemassen für die Beispiele 3 und 4.** | | | | |
|---|---|---|---|---|
| Beispiel | Polymer | Polystyrol (%) | Bupranolol (%) | Maximum Tangens delta^{a} (°C) |
| 3a | Kraton GX | 14 | 0 | 7,8 |
| 3b | 1657 | | 2,5 | 8,2 |
| 3c | | | 5 | 7,3 |
| 3d | | | 10 | 7,6 |
| 3e | | | 20 | 7,5 |
| | | | | |
| 4a | Cariflex TR | 15 | 0 | 7,4 |
| 4b | 1107 | | 2,5 | 8,0 |
| 4c | | | 5 | 7,3 |
| 4d | | | 10 | 7,0 |
| 4e | | | 20 | 5,1 |

| | | | | |
|---|---|---|---|---|
| ^{a} Messfrequenz 1Hz. | | | | |

Die Temperatur, bei der der Tangens delta ein Maximum erreicht wurde für Cariflex TR 1107 und Kraton GX 1657 bestimmt. Die Ergebnisse sind in Tabelle 8 dargestellt.

### Charakterisierung des Endblock-Temperaturbereiches

Die wirkstofffreien bzw. wirkstoffbeladenen Klebemassen wurden mit Hilfe der dynamisch-mechanischen Analyse charakterisiert. Die Wirkstoffbeladung betrug 2,5, 5, 10 und 20% Bupranolol. Die Bestimmung des dynamisch-mechanischen Verhaltens im Temperaturbereich der Anwendungstemperatur (32°C) und des Polystyrol-Glasüberganges, erfolgte mit einem Rheometrics RDS 7700. Als Steuergerät wurde ein PC verwendet, der mit der Software RHIOS 3.01 betrieben wurde. Es wurde im Parallel-Platten-Modus gearbeitet. Der Plattendurchmesser betrug 25 mm. Die Frequenz der sinusförmigen Anregung betrug 1 Hz bzw. 6.28 rad/s. Der erfasste Temperaturbereich lag zwischen 25 und 130 °C. Die Temperatur wurde in Schritten von 6° C erhöht. Ausgangstemperatur war 25°C. Die Temperaturausgleichszeit der Probe betrug 90 s. Der Tangens delta (Dämpfung), der Verlust- und Schubmodul wurden bestimmt.

**Tabelle 9**

| **Temperatur, bei der das Schubmodul (G') unter einen Wert 10000 Pa abfällt. Messwerte für die Beispiele 3 und 4.** | | | | |
|---|---|---|---|---|
| Beispiel | Polymer | Polystyrolanteil Gew.-% | Bupranolol Gew.-% | Temperatur (°C) G'<10000 Pa |
| 3a | Kraton GX | 14 | 0 | 82 |
| 3b | 1657 | | 2,5 | 77 |
| 3c | | | 5 | 74 |
| 3d | | | 10 | 71 |
| 3e | | | 20 | 62 |
| | | | | |
| 4a | Cariflex TR | 15 | 0 | 63 |
| 4b | 1107 | | 2,5 | 62 |
| 4c | | | 5 | 58 |
| 4d | | | 10 | 56 |
| 4e | | | 20 | 32 |

Der Vergleich der in der Tabelle 9 aufgeführten Messwerte zeigt, dass die Temperatur, bei der das Schubmodul unter 10 000 Pa absinkt, bei vergleichbaren Polystyrolgehalten mit steigender Wirkstoffbeladung unterschiedlich stark abfällt. Im Bereich von 10 000 Pa geht das Klebesystem in den Zustand einer Schmelze über. Der Abstand zwischen der Anwendungstemperatur und der Temperatur dieses Ueberganges gibt einen Anhaltspunkt für die Eignung der Klebemasse als Haftklebstoff Der Wert des wirkstofffreien Cariflex TR 1107 liegt im Bereich des mit 20% Gew.-% Bupranolol beladenen Kraton GX 1657.

Während der Temperaturabfall für GX 1657 mit gesättigtem Mittelblock bei steigender Wirkstoffbeladung annähernd linear abfällt, ist bei TR 1107 mit mehr als 10 Gew.-% Bupranolol überraschenderweise ein starker Abfall zu beobachten. Der Temperaturabfall ist für das auf TR 1107 basierende Klebersystem mit 20% Gew.-% Bupranolol so weitgehend, dass im Bereich der Anwendungstemperatur keine für ein Haftklebesystem notwendige Kohäsivität mehr vorhanden ist. Die Kohäsivität ist dabei soweit erniedrigt, dass das Klebersystem sich einerseits von der Trägerschicht ablöst und andererseits eine Ablösung des Klebesystem von der Haut zum Verbleiben von massiven Rückständen der Klebemasse auf der Haut führt.

**Tabelle 10**

| **Schubmodule (G') von Klebemassen auf Basis von GX 1657 bei Hauttemperatur (32°C). Die Bestimmung erfolgte im Parallel-Platten-Modus. Der Plattendurchmesser betrug 25 mm.** | | | | |
|---|---|---|---|---|
| Beispiel | Polymer | Polystyrol Gew.-% | Bupranolol Gew.-% | Schubmodul bei 32°C (Pa) |
| 3a | Kraton GX | 14 | 0 | 1,14 E5 |
| 3b | 1657 | | 2,5 | 9,53 E4 |
| 3c | | | 5 | 1,02 E5 |
| 3d | | | 10 | 8,34 E4 |
| 3e | | | 20 | 5,32 E4 |
| | | | | |
| 4a | Cariflex TR | 15 | 0 | 3,07 E4 |
| 4b | 1107 | | 2,5 | 3,08 E4 |
| 4c | | | 5 | 2,29 E4 |
| 4d | | | 10 | 2,46 E4 |
| 4e | | | 20 | 9,30 E3 |

In Tabelle 10 sind die Ergebnisse der dynamisch-mechanischen Charakterisierung für den Schubmodul bei Hauttemperatur (32°C) dargestellt.Unter Berücksichtigung der Literatur (Satas,D. (Ed.), Handbook of pressure-sensitive adhesive technology, Van Nostrand Reinhold, New York, S. 158 ff, 1989) sind gute Klebeleistungen zu erwarten, wenn der Schubmodul (G') bei der Anwendungstemperatur zwischen 50 000 und 200 000 Pa liegt. In Tabelle 8 sind die Ergebnisse der dynamisch-mechanischen Charakterisierung aufgeführt. Während die Messwerte zeigen, dass die Rezeptur auf der Basis von Kraton GX 1657 im Bereich dieser Grenzen liegt, liegen die Messwerte für die Rezeptur auf der Basis von TR 1107 deutlich unter dem Wert von 50 000 Pa. Bei einem Beladungsgrad von 20% Bupranolol lassen sich mit mit Kraton GX 1657 nach dem ausgewählten Rezepturbeispiel Trägersysteme herstellen, die den Anforderungen an die viskoelastischen Eigenschaften eines Wirkstoffpflasters gerecht werden. Bei Trägersystemen auf der Basis von Cariflex TR 1107 lassen sich für keines der aufgeführten Rezepturbeispiele viskoelastische Eigenschaften im Bereich der Anwendungstemperatur feststellen, die den Anforderungen an ein haftklebendes Reservoir gerecht werden.

### Beispiele 5a, b und 6a, b

### Herstellung der Klebemassen

### Beispiele 5a, b

Kraton G 1657 (SEBS-Dreiblockcopolymer ) oder Cariflex TR 1107 (SIS-Dreiblockcopolymer), Regalrez 1094 (aliphatisches Kohlenwasserstoffharz), Abitol (Tackifier) werden in einem Laborkneter bei 160°C in der angegebenen Menge (s. Tab. 11) aufgeschmolzen und bis zur Homogenität gemischt (Dauer ca 60 Minuten). Dieser Vorgang findet ohne Luftzutritt statt, eine Schutzgasatmosphäre wird nicht eingesetzt. Der klaren Schmelze wird Bupranolol zugesetzt. Anschliessend wird unter grossflächigem Luftzutritt weiter gemischt bzw. geknetet. Nach 120, 180, 240, 300 und 360 min werden Proben von ca 10 g aus dem knettrog zur Molekulargewichtsbestimmung entnommen.

**Tabelle 11**

| **Zusammensetzung Beispiel 5a und b.** Mengenangaben in g | | | | | |
|---|---|---|---|---|---|
| Beispiel | Cariflex TR | Kraton GX | Regalrez Abitol Bupranolol | | |
| | #1107 | #1657 | #1094 | | |
| 5a | 112,50 | | 75,00 | 50,00 | 12,50 |
| 5b | | 112,50 | 75,00 | 50,00 | 12,50 |

### Beispiele 6a, b

Kraton G 1657 (SEBS-Dreiblockcopolymer ) oder Cariflex TR 1107 (SIS-Dreiblockcopolymer), Regalrez 1094 (aliphatisches Kohlenwasserstoffharz), Abitol (Tackifier) und Irganox 1010 (Antioxidans) werden in einem Laborkneter bei 160°C, unter Argon, in der angegebenen Menge (s. Tab. 12) aufgeschmolzen und bis zur Homogenität gemischt (Dauer ca 60 Minuten). Der klaren Schmelze wird Bupranolol zugesetzt. Anschliessend wird unter Luftabschluss und Argon-Atmosphere weitergeknetet. Nach 120, 180, 240, 300 und 360 min werden Proben von ca 10 g aus dem Knettrog entnommen. Das Molekulargewicht der Proben wird mit GPC ermittelt.

**Tabelle 12**

| **Zusammensetzung Beispiel 6a und b.** Mengenangaben in g | | | | | | |
|---|---|---|---|---|---|---|
| Beispiel | Cariflex TR | Kraton GX | Regalrez | Irganox | Abitol | Bupranolol |
| | #1107 | #1657 | #1094 | #1010 | | |
| 6a | 112,50 | | 75,00 | 1,25 | 50,00 | 12,50 |
| 6b | | 112,50 | 75,00 | 1,25 | 50,00 | 12,50 |

### Molekulargewichtsbestimmung

Die Molekulargewichtsbestimmungen erfolgten mittels Gelpermeationschromatographie. Die verwendet Anlage bestand aus einer Lichrograph L-6000 HPLC-Pumpe (Merck, D-Darmstadt), einem Säulenthermostat T-6300 (Merck), einem ERC-7512 Brechungsindex-Detektor (Erma, J-Tokyo) und einem D-2520 GPC Integrator (Merck). Es wurde eine Polymer Laboratories (UK-Shropshire) PL-Gel 5µ Mix Säule verwendet. Die Säule war 300 mm lang, der innere Durchmesser betrug 7,5 mm; die Partikelgrösse der Säulenfüllung betrug 5 µm. Die Säuleneichung erfolgte mit Polystyrol, dazu wurde ein Polymer Laboratories Mole Standard: Polystyrene-medium molecular weight calibration kit verwendet. Als Laufmittel diente Tetrahydrofuran. Die Säulentemperatur betrug 35°C, der Druck 25 bar; die Fliessgeschwindigkeit des Laufmittels war auf 1 ml/min eingestellt.

Die Proben wurden in Tetrahydrofuran gelöst und mit einer entsprechenden Menge Toluol versetzt.

**Tabelle 13**

| **Beispiel 5 a. Zahlenmittlere, gewichtsmittlere und z-mittlere Molmasse und Polydispersität von Klebemassen auf der Basis von Cariflex TR 1107 (*****SIS-Dreiblockcopolymer*****), die zwischen 120 und 360 min im Laborkneter wärmebehandelt wurden. Die Klebemasse wurde nicht mit Irganox oder Argon stabilisiert.** | | | | |
|---|---|---|---|---|
| Probennahme | Mₙ | M_{w} | M_{z} | M_{w}/Mₙ |
| unbehandelt | 159048 | 197247 | 239912 | 1,240 |
| 120 min | 61806 | 114799 | 176420 | 1,857 |
| 180 min | 46679 | 84497 | 133398 | 1,810 |
| 240 min | 39377 | 69338 | 109044 | 1,760 |
| 300 min | 32701 | 56307 | 88183 | 1,721 |
| 360 min | 28134 | 46600 | 71924 | 1,656 |

**Tabelle 14**

| **Beispiel 5 b. Zahlenmittlere, gewichtsmittlere und z-mittlere Molmasse und Polydispersität von Klebemassen auf der Basis von Kraton GX 1657 (*****SEBS-Dreiblockcopolymer*****), die zwischen 120 und 360 min im Laborkneter wärmebehandelt wurden. Die Klebemasse wurde nicht mit Irganox oder Argon stabilisiert.** | | | | |
|---|---|---|---|---|
| Probennahme | Mₙ | M_{w} | M_{z} | M_{w}/Mₙ |
| unbehandelt | 98396 | 121378 | 142518 | 1,233 |
| 120 min | 93791 | 117405 | 144724 | 1,251 |
| 180 min | 96591 | 119772 | 141034 | 1,239 |
| 240 min | 97425 | 121307 | 142906 | 1,245 |
| 300 min | 95941 | 119673 | 141184 | 1,247 |
| 360 min | 94705 | 118409 | 139956 | 1,250 |

**Tabelle 15**

| **Beispiel 6a. Zahlenmittlere, gewichtsmittlere und z-mittlere Molmasse und Polydispersität von Klebemassen auf der Basis von GX 1657, die zwischen 120 und 360 min im Laborkneter wärmebehandelt wurden. Die Klebemasse wurde mit Irganox und Argon stabilisiert.** | | | | |
|---|---|---|---|---|
| Probennahme | Mₙ | M_{w} | M_{z} | M_{w}/Mₙ |
| 120 min | 93080 | 163146 | 223606 | 1,752 |
| 180 min | 91739 | 162362 | 223376 | 1,769 |
| 240 min | 92601 | 165590 | 230121 | 1,788 |
| 300 min | 90402 | 161962 | 227359 | 1,791 |
| 360 min | 90922 | 163664 | 228981 | 1,800 |

**Tabelle 16**

| **Beispiel 6b. Zahlenmittlere, gewichtsmittlere und z-mittlere Molmasse und Polydispersität von Klebemassen auf der Basis von GX 1657, die zwischen 120 und 360 min im Laborkneter wärmebehandelt wurden. Die Klebemasse wurde mit Irganox und Argon stabilisiert.** | | | | |
|---|---|---|---|---|
| Probennahme | Mₙ | M_{w} | M_{z} | M_{w}/Mₙ |
| 120 min | 93952 | 116246 | 136825 | 1,237 |
| 180 min | 93569 | 115310 | 135493 | 1,232 |
| 240 min | 94924 | 116940 | 137383 | 1,231 |
| 300 min | 94536 | 117397 | 139381 | 1,241 |
| 360 min | 93866 | 116185 | 137083 | 1,237 |

In Tabelle 13 und 14 sind die Veränderungen der Molmassenverteilung mit entsprechenden Parametern für die Beispiele 5a und 5b dargestellt. Bei der auf Cariflex TR 1107 basierenden, unstabilisierten-Klebemasse kommt es zu einem erheblichen Polymerabbau während der Wärmebehandlung, der durch die Verschiebung der Molmassenverteilung zu kleineren Molekulargewichten gekennzeichnet ist. Bei der unstabilisierten Klebemasse auf der Basis von GX 1657 ist die Veränderung erheblich kleiner. Vergleicht man die stabilisierten Beispiele 6a und 6b (Tab. 15 und 16) miteinander, fällt auf, dass auch hier die Klebemasse auf der Basis von GX 1657 erheblich geringere Veränderungen erfährt. Überraschenderweise ist auch die stabilisierte Rezeptur, Beispiel 6a, wesentlich anfälliger für einen Polymerabbau, insbesondere, wenn man die Molmassenverteilungen der unbehandelten Polymere zum Vergleich heranzieht.

Rezepturen auf der Basis von Polymeren, die einen gesättigten Mittelblock enthalten, zeigen ein höhere Stabilität während der Verarbeitung im Hotmelt-Verfahren. Die Menge an notwendigen Stabilisatoren kann gegenüber den Vergleichspolymern mit ungesättigtem Mittelblock weitgehend reduziert werden. Da auch Stabilisatoren und deren Derivate als potentiell hautreizend angesehen werden müssen, ergeben sich diesbezüglich Vorteile für die erfindungsgemässe Zusammensetzung des Haftschmelzklebers.

### Beispiel 7

### Nikotinpflaster

1039,5 g Kraton G 1657 (SEBS-Dreiblockcopolymer) und 16,5 g Irganox 1010 werden in einem Kneter auf 170°C aufgeheizt (Dauer ca 45 Minuten). Anschließend werden nacheinander portionsweise 1419 g Regalrez 1094 (aliphatisches Kohlenwasserstoffharz) und 561 g Abitol (Hydroabiethylalkohol) bis zur Homogenität zugemischt (Dauer ca 240 Minuten). In der klaren Schmelze werden unter Inertgas bei 150°C 299,5 g Nikotin durch tropfenweise Zugabe gelöst (Dauer ca 30 Minuten). Die erhaltene, 150°C heiße nikotinhaltige Haftschmelzklebmasse wird kontinuierlich durch eine Schlitzdüse gepreßt und mit einer Geschwindigkeit von 5 m/min in einer Dicke von ca 150 µm auf eine gekühlte, silikonisierte Polyesterfolie (Schutzschicht) aufgebracht. Auf die offenliegende Haftklebefläche wird unter Kühlung eine 15 µm dicke Polyesterfolie (Rückschicht) laminiert.

Aus dem erhaltenen Laminat werden 16 cm² große Einzelpflaster ausgestanzt.

### Vergleichsbeispiel 7'

### Placebopflaster

Die Herstellung erfolgt gemäß Beispiel 5, jedoch ohne Zusatz von Nikotin.

### Dynamisch-mechanische Analyse

### Bestimmung des Elastizitätsmoduls

Das elastische Modul G' der gemäß Beispiel 7 und 7' hergestellten Nikotin- und Placebopflaster wurde mit Hilfe des DMTA Gerätes, Modell Eplexor (Fa. Gabo) in Abhängigkeit von der Temperatur bestimmt. Die Messung erfolgte im Schermodus an 14∗14 mm großen Proben, bestehend aus Klebefilm und Rückschicht, entsprechend DIN 53513 mit einer Frequenz von 10 Hz. Der erfaßte Temperaturbereich lag zwischen - 50 und 80°C, wobei die Temperatur ausgehend von - 50°C in Schritten von 1°C erhöht wurde. Nach Temperaturausgleich der Probe wurde jeweils das zugehörige Schubmodul G' bestimmt.

Das so bestimmte Modul G' lag bei 32°C, d.h. im Hauttemperaturbereich, sowohl beim wirkstoffhaltigen als auch wirkstofffreien Pflaster einheitlich bei 1,1 E5 Pa. Danach hatte sich diese für die Beurteilung der Klebeeigenschaften des Pflasters wichtige Kenngröße trotz einer relativ hohen (ca 8 %-igen) Beladung des Haftschmelzklebers mit Nikotin nicht geändert.

### Vergleichsbeispiel 8

### Nikotinpflaster

### Herstellung aus der Lösung

Eine nikotinhaltige Haftklebermasse bestehend aus
- 170 g: Nikotin
- 350 g: Cariflex TR 1107 ( Polystyrol-polyisopren-polystyrol-Dreiblockcopolymer)
- 350 g: Hercurez C (aliphatisches Kohlenwasserstoffharz)
- 280 g: Abitol (Hydroabiethylalkohol)
- 450 g: Elcema P050 (Zellulose zur Bindung von Nikotin)
- 1.050 g: Spezialbenzin 80-110 als Lösungsmittel
wird so auf eine ca 100 µm dicke, silikonisierte Schutzfolie aufgetragen, daß nach Entfernen des Lösungsmittels eine Haftkleberschicht von ca.77,75 g/m² resultiert. Zwei dieser Kleberschichten werden bei gleichzeitigem Austausch einer der Schutzschichten gegen eine 20 µm dicke Polyesterfolie aufeinanderkaschiert, so daß ein Nikotinpflaster mit einem Klebefilm von ca. 155,5 g/m² erhalten wird.

Aus dem erhaltenen Laminat werden 16 cm² große Einzelpflaster ausgestanzt.

### Wirkstofffreisetzung

Die Messung der Nikotinfreisetzung von Beispiel 7 und Vergleichs-beispiel 8 wird nach der USP XXII Paddle-over-Disk-Methode in Wasser bei 32°C durchgeführt. Die pro 16 cm² nach 1, 2 und 3 Std. freigesetzten Mengen an Nikotin werden flüssigkeitschromatographisch bestimmt. Das Ergebnis der Untersuchung ist in Tabelle 17 aufgeführt. Wie die Meßwerte zeigen, ist die Freisetzung des leicht flüchtigen Nikotins aus dem erfindungsgemäßen Reservoir deutlich stärker retardiert als bei dem Vergleichsbeispiel.

**Tabelle 17**

| **Nikotinfreisetzung** | | | |
|---|---|---|---|
| Testpräparat | Mittlere Freisetzung in mg/16 cm² nach | | |
| | 1 h | 2 h | 3 h |
| Beispiel 7 ( n = 3 ) | 3,4 | 4,8 | 5,8 |
| Vergleichsbeispiel 8 ( n = 6 ) | 9,2 | 12,7 | 15,8 |

## Patentansprüche

1. Wirkstoffpflaster zur kontrollierten Abgabe von Wirkstoffen an die Haut, bestehend aus einer Ruckschicht, einem damit verbundenen, wasserunlöslichen Klebefilm aus einem wirkstoffhaltigen Haftschmelzkleber, als Matrixschicht ausgestaltet, und einer den Klebefilm abdekkenden, wieder ablösbaren Schicht, dadurch gekennzeichnet, daß die Matrixschicht einen Haftschmelzkleber ohne Zusatz von in Wasser quellbaren Polymeren, der ein chemisch gesättigtes Dreiblockcopolymer aus Polystyrolblock-copoly(ethylen-butylen)-block-poly-styrol (SEBS) ist, in einer Konzentration von 10 bis 80 Gew.-% und einen bei der Verarbeitungstemperatur des Haftschmelzklebers flüssigen, niedrigschmelzenden und/oder flüchtigen Wirkstoff in einer Konzentration von 2,5 bis 25 Gew.-% enthält.

2. Wirkstoffpflaster nach Anspruch 1, dadurch gekennzeichnet, daß es bevorzugt das Dreiblockcopolymer (SEBS) in einer Konzentration von 20 bis 40 Gew.-% enthält.

3. Wirkstoffpflaster nach Ansprüchen 1 und 2, dadurch gekennzeichnet, daß der Styrolgehalt des SEBS-Dreiblockcopolymeren 10 bis 50 Gew.-% beträgt.

4. Wirkstoffpflaster nach Ansprüchen 1-3, dadurch gekennzeichnet, daß der Styrolgehalt des SEBS-Dreiblockcopolymeren bevorzugt 10 bis 30 Gew.-% beträgt.

5. Wirkstoffpflaster nach Ansprüchen 1 bis 4, dadurch gekennzeichnet, daß der Haftschmelzkleber ein Dreiblockcopolymer aus Polystyrol-block-copoly(ethylenbutylen)-block-poly-styrol (SEBS) in einer Konzentration von 10 bis 80 Gew.-% ist, zwischen 20 und 90 Gew.-% Klebrigmacher (Tackifier) und gegebenenfalls 0,1 bis 1 Gew.-% Alterungsschutzmittel enthält.

6. Wirkstoffpflaster nach Anspruch 5, dadurch gekennzeichnet, daß der Haftschmelzkleber bevorzugt 40 bis 70 Gew.-% Klebrigmacher (Tackifier) und gegebenenfalls 0,1 bis 1 Gew.-% Alterungsschutzmittel enthält.

7. Wirkstoffpflaster nach Ansprüchen 5 und 6, dadurch gekennzeichnet, daß der Haftschmelzkleber als Tackifier aliphatische und/oder aromatische Kohlenwasserstoffharze enthält, die mit den Endblöcken und/oder dem Mittelblock des SEBS-Dreiblock-polymeren kompatibel sind.

8. Wirkstoffpflaster nach Ansprüchen 5 bis 7, dadurch gekennzeichnet, daß der Haftschmelzkleber als Tackifier Hydroabiethylalkohol enthält.

9. Wirkstoffpflaster nach Ansprüchen 1 bis 8, dadurch gekennzeichnet, daß es als Wirkstoff Nikotin enthält.

10. Wirkstoffpflaster nach Ansprüchen 1 bis 9, dadurch gekennzeichnet, daß es als Wirkstoff ein β-Rezeptorenblocker enthält.

11. Verfahren zur Herstellung von Wirkstoffpflastern nach Ansprüchen 1 bis 10, dadurch gekennzeichnet, daß die die Matrixschicht bildenden Bestandteile des Haftschmelzklebers vor Zugabe des Wirkstoffes unter Erwärmen auf 100 bis 200°C in einer Inertgasatmosphäre bis zum Erhalt einer homogenen Schmelze gemischt werden und der Wirkstoff anschließend unter Inertgas in der Haftkleberschmelze bei einer Verarbeitungstemperatur von 100 bis 200°C aufgelöst wird, und die homogene Mischung auf die wirkstoffundurchlässige Deckschicht aufgebracht wird.

12. Verfahren zur Herstellung von Wirkstoffpflastern nach Ansprüchen 1 bis 11, dadurch gekennzeichnet, daß die Bestandteile des Haftschmelzklebers vor Zugabe des Wirkstoffes unter Erwärmen auf bevorzugt 110 bis 170°C in einer Inertgasatmosphäre bis zum Erhalt einer homogenen Schmelze gemischt werden und der Wirkstoff anschließend unter Inertgas in der Haftkleberschmelze bei einer Verarbeitungstemperatur von bevorzugt 110 bis 130°C aufgelöst wird.

13. Verfahren zur Herstellung von Wirkstoffpflastern nach Ansprüchen 1-12, dadurch gekennzeichnet, daß die homogene wirkstoffhaltige Haftschmelzklebemasse auf die wieder ablösbare Schutzschicht oder eine antiadhäsive Grundlage durch Extrusion, Gießen, Walzenauftrag, Rakelauftrag, Aufsprühen oder ein Druckverfahren aufgebracht wird und mit der Rückschicht abgedeckt wird.

14. Verfahren zur Herstellung von Wirkstoffpflastern nach Ansprüchen 1-13, dadurch gekennzeichnet, daß die homogene wirkstoffhaltige Haftschmelzklebemasse auf die Rückschicht durch Extrusion, Gießen, Walzenauftrag, Rakelauftrag, Aufsprühen oder ein Druckverfahren aufgebracht wird und mit der wieder ablösbaren Schutzschicht abgedeckt wird.

15. Verfahren zur Herstellung von Wirkstoffpflastern nach Ansprüchen 1-14, dadurch gekennzeichnet, daß die Vereinzelung der Wirkstoffpflaster durch Schneiden und/oder Formatstanzen erfolgt.

## Claims

1. Active ingredient patch, for the controlled release of active ingredients to the skin consisting of a backing layer, and, bonded to it, of a water-insoluble adhesive film consisting of an active-ingredient-containing contact hot-melt adhesive organized as a matrix layer, and of a removable layer covering the adhesive film, characterized by the fact that the matrix layer comprises a contact hot-melt adhesive, which is a chemically saturated three-block copolymer of polystyrene-block-copoly-(ethylene-butylene)-block-polystyrene (SEBS), in a concentration of 10 to 80 weight % and without the addition of water-swellable polymers, and the matrix layer further comprises an active ingredient, at a concentration of 2.5 to 25 weight %, which is liquid, low-melting and/or volatile at the processing temperature of the contact hot-melt adhesive.

2. Active ingredient patch according to Claim 1, characterized by the fact that it preferably contains a three-block copolymer (SEBS) in a concentration of 20 to 40 weight %.

3. Active ingredient patch according to Claims 1 and 2, characterized by the fact that the styrene content of the SEBS three-block copolymer is 10 to 50 weight %.

4. Active ingredient patch according to Claims 1-3, characterized by the fact that the styrene content of the SEBS three-block copolymer is preferably 10 to 30 weight %.

5. Active ingredient patch according to Claims 1-4, characterized by the fact that the contact hot-melt adhesive is a three-block copolymer of polystyrene-block-copoly (ethylene-butylene)-block-polystyrene (SEBS), at a concentration of 10 to 80 weight % and contains between 20 and 90 weight % tackifier and optionally 0.1 to 1 weight % antiaging agent.

6. Active ingredient patch according to Claim 5, characterized by the fact that the contact hot-melt adhesive contains preferably 40 to 70 weight % tackifier and optionally 0.1 to 1 weight % antiaging agent.

7. Active ingredient patch according to Claims 5 and 6, characterized by the fact that the contact hot-melt adhesive contains aliphatic and/or aromatic hydrocarbon resins as tackifier which are compatible with the end blocks and/or middle block of the SEBS three-block-polymer.

8. Active ingredient patch according to Claims 5-7, characterized by the fact that the contact hot-melt adhesive contains hydroabietyl alcohol as tackifier.

9. Active ingredient patch according to Claims 1-8, characterized by the fact that it contains nicotine as active ingredient.

10. Active ingredient patch according to Claims 1-9, characterized by the fact that it contains a β-receptor blocker as active ingredient.

11. Method for the preparation of active ingredient patches according to Claims 1-10, characterized by the fact that the components of the contact hot-melt adhesive that form the matrix layer are mixed under heating to 100 to 200°C in an inert gas atmosphere until a homogeneous melt is obtained, before adding active ingredient, and then the active ingredient is dissolved in the contact adhesive melt under an inert gas at a processing temperature of 100 to 200°C and the homogeneous mixture is applied onto the covering layer, which is impermeable to the active ingredient.

12. Method for the preparation of active ingredient patches according to Claims 1-11, characterized by the fact that the components of the contact hot-melt adhesive are mixed before the addition of the active ingredient by heating to preferably 110 to 170°C in an inert gas atmosphere until a homogeneous melt is obtained and then the active ingredient is dissolved in the contact adhesive melt under an inter gas at a processing temperature of preferably 110 to 130°C.

13. Method for the preparation of active ingredient patches according to Claims 1-12, characterized by the fact that the homogeneous active-ingredient-containing contact hot-melt adhesive composition is applied onto the removable protective layer or onto an antiadhesive substrate by extrusion, casting, roll application, blade application, spraying or by a pressure process and is covered with the backing layer.

14. Method for the preparation of active ingredient patches according to Claims 1-13, characterized by the fact that the homogeneous active-ingredient-containing contact hot-melt adhesive composition is applied onto the backing layer by extrusion, casting, roll application, blade application, spraying or a pressure method and is covered with the removable protective layer.

15. Method for the preparation of active ingredient patches according to Claims 1-14, characterized by the fact that the division of the active ingredient patch is done by cutting and/or format stamping.

## Revendications

1. Emplâtre à principe actif pour la libération contrôlée de principes actifs à la peau, constitué d'une couche dorsale, d'un film adhésif insoluble dans l'eau, relié à celle-ci, formé d'une colle de contact fusible contenant un principe actif, conçu sous forme de couche matricielle, et d'une couche détachable recouvrant le film adhésif, caractérisé en ce que la couche matricielle contient une colle de contact fusible sans addition de polymères gonflables dans l'eau, qui est un copolymère triséquencé chimiquement saturé de polystyrène-copoly(éthylène-butylène)-polystyrène (SEBS), en une concentration de 10 à 80 % en poids et un principe actif liquide, à bas point de fusion et/ou volatile à la température de mise en oeuvre de la colle de contact fusible, en une concentration de 2,5 à 25 % en poids.

2. Emplâtre à principe actif selon la revendication 1, caractérisé en ce qu'il contient de préférence le copolymère triséquencé (SEBS) en une concentration de 20 à 40 % en poids.

3. Emplâtre à principe actif selon les revendications 1 et 2, caractérisé en ce que la teneur en styrène du copolymère triséquencé de SEBS vaut 10 à 50% en poids.

4. Emplâtre à principe actif selon les revendications 1 à 3, caractérisé en ce que la teneur en styrène du copolymère triséquencé de SEBS vaut de préférence 10 à 30 % en poids.

5. Emplâtre à principe actif selon les revendications 1 à 4, caractérisé en ce que la colle de contact fusible est un copolymère triséquencé de polystyrène-copoly(éthylène-butylène)-polystyrène (SEBS), en une concentration de 10 à 80 % en poids, contient 20 à 90 % en poids d'agent adhésif (tackifier) et éventuellement 0,1 à 1 % en poids d'agent de protection contre le vieillissement.

6. Emplâtre à principe actif selon la revendication 5, caractérisé en ce que la colle de contact fusible contient de préférence 40 à 70 % en poids d'agent adhésif (tackifier) et éventuellement 0,1 à 1 % en poids d'agent de protection contre le vieillissement.

7. Emplâtre à principe actif selon les revendications 5 et 6, caractérisé en ce que la colle de contact fusible contient à titre de tackifier des résines hydrocarbonées aliphatiques et/ou aromatiques, qui sont compatibles avec les unités de séquence terminales et/ou l'unité de séquence centrale du polymère triséquencé de SEBS.

8. Emplâtre à principe actif selon les revendications 5 à 7, caractérisé en ce que la colle de contact fusible contient à titre de tackifier de l'alcool hydroabiéthylique.

9. Emplâtre à principe actif selon les revendications 1 à 8, caractérisé en ce qu'il contient de la nicotine à titre de principe actif.

10. Emplâtre à principe actif selon les revendications 1 à 9, caractérisé en ce qu'il contient un bloqueur des récepteurs β à titre de principe actif.

11. Procédé de préparation d'emplâtres à principe actif selon les revendications 1 à 10, caractérisé en ce que les constituants de la colle de contact fusible formant la couche matricielle sont mélangés avant l'addition du principe actif par chauffage à 100 à 200 °C dans une atmosphère de gaz inerte jusqu'à l'obtention d'une masse fondue homogène et en ce que le principe actif est ensuite solubilisé sous gaz inerte dans la colle de contact fondue à une température de mise en oeuvre de 100 à 200 °C et en ce que le mélange homogène est appliqué sur la couche de recouvrement imperméable au principe actif.

12. Procédé de préparation d'emplâtres à principe actif selon les revendications 1 à 11, caractérisé en ce que les constituants de la colle de contact fusible sont mélangés avant l'addition du principe actif par chauffage de préférence à 110 à 170 °C dans une atmosphère de gaz inerte jusqu'à l'obtention d'une masse fondue homogène et en ce que le principe actif est ensuite solubilisé sous gaz inerte dans la colle de contact fondue à une température de mise en oeuvre de préférence de 110 à 130 °C.

13. Procédé de préparation d'emplâtres à principe actif selon les revendications 1 à 12, caractérisé en ce que la colle de contact fusible homogène contenant le principe actif est appliquée sur la couche de protection détachable ou sur une base anti-adhésive par extrusion, par moulage, par application au rouleau, par application à la lame, par projection ou par un procédé d'impression et recouverte de la couche dorsale.

14. Procédé de préparation d'emplâtres à principe actif selon les revendications 1 à 13, caractérisé en ce que la colle de contact fusible homogène contenant le principe actif est appliquée sur la couche dorsale par extrusion, par moulage, par application au rouleau, par application à la lame, par projection ou par un procédé d'impression et recouverte de la couche de protection détachable.

15. Procédé de préparation d'emplâtres à principe actif selon les revendications 1 à 14, caractérisé en ce que l'individualisation des emplâtres à principe actif est effectuée par sectionnement et/ou par découpage au format.
